Europäisches Patentamt

European Patent Office

Office européen des brevets

(19)

(11) Veröffentlichungsnummer: **0 431 481 A1**

(12) **EUROPÄISCHE PATENTANMELDUNG**

(21) Anmeldenummer: **90122911.2**

(22) Anmeldetag: **30.11.90**

(51) Int. Cl.5: **G01N 33/36**

(30) Priorität: **04.12.89 CH 4330/89**

(43) Veröffentlichungstag der Anmeldung:
**12.06.91 Patentblatt 91/24**

(84) Benannte Vertragsstaaten:
**CH DE ES FR GB IT LI**

(71) Anmelder: **MASCHINENFABRIK RIETER AG**

**CH-8406 Winterthur(CH)**

(72) Erfinder: **Demuth, Robert**
**Maulackerstrasse 17**
**CH-8309 Nürensdorf(CH)**
Erfinder: **Faas, Jürg**
**Seuzacherstrasse 16**
**CH-8474 Dinhard(CH)**
Erfinder: **Moser, Robert**
**Wingertlistrasse 41**
**CH-8405 Winterthur(CH)**

(54) **Verfahren zur kontinuierlichen Ermittlung der Faserfeinheit von Fasern in Bändern und entsprechende Vorrichtung zur Durchfürung des Verfahrens.**

(57) Das Verfahren dient dazu, Messdaten, die aus kontinuierlichen Messungen stammen, die von mehreren Parametern, insbesondere Eigenschaftsparametern von Faserbändern und Fasern, aus denen die Faserbänder bestehen, und auch von Umgebungs-, Verarbeitungs, und Messparametern abhängig sind, durch Kalibrierung und kombinierte Verarbeitung umzurechnen in Daten, die nur einzelne Eigenschaftsparameter und ihre zeitlichen Veränderungen betreffen und in bezug auf Umgebungs- und Verarbeitungsparameter auf Standardbedingungen reduziert sind. Die entsprechende Vorrichtung besteht aus zwei Sensoren, einer Kalibrier- und einer Verarbeitungseinheit und erlaubt es, kontinuierlich die Materialmenge pro Bandlänge des betreffenden Faserbandes und die zeitlichen Veränderungen der Faserfeinheit zu messen.

FIG. 3B

EP 0 431 481 A1

# VERFAHREN ZUR KONTINUIERLICHEN ERMITTLUNG DER FASERFEINHEIT VON FASERN IN BÄNDERN UND ENTSPRECHENDE VORRICHTUNG ZUR DURCHFUEHRUNG DES VERFAHRENS.

Die Erfindung liegt auf dem Gebiete der Textiltechnik und betrifft ein Verfahren zur Messung der Faserfeinheit in Faserbändern gemäss dem Oberbegriff des unabhängigen Verfahrensanspruches und eine Vorrichtung gemäss dem Oberbegriff des unabhängigen Vorrichtungsanspruches, mit welcher kontinuierlich die Faserfeinheit in einem Faserband, das sich relativ zur Vorrichtung bewegt, und die Schwankungen in der Faserfeinheit der das Faserband bildenden Fasern ermittelt werden können.

Die Aufgabe der Öffnerei und des Spinnereivorwerkes ist es, kontinuierlich Faserbänder herzustellen, die in allen ihren Eigenschaften möglichst kleinen Schwankungen unterworfen sind und vorgegebenen Sollwerten möglichst entsprechen. Diese Aufgabe kann nur mit Hilfe einer kontinuierlichen Kontrolle und Korrektur an verschiedenen Stellen des Prozessablaufes optimal gelöst werden. Dazu müssen kontinuierlich Daten über die Eigenschaften des Faserbandes an verschiedenen Stellen des Verarbeitungsprozesses zur Verfügung stehen.

Die Eigenschaften eines Faserbandes, wie es im Spinnereivorwerk von Stufe zu Stufe für die Spinnerei vorbereitet wird, sind abhängig von Eigenschaften der Fasern, aus denen das Faserband besteht, von Art und Stufe der Verarbeitung und von Umgebungsbedingungen. Die Eigenschaften eines bestimmten Faserbandes sind also bestimmt durch ein dem Faserband entsprechendes Set von Parameterwerten. Die wichtigsten Parameter, die ein solches Set ausmachen sind

Faserbandparameter:
- die Provenienz der Fasern,
- die Menge Textilmaterial pro Längeneinheit Faserband,
- die gegenseitige Lage der das Faserband bildenden Fasern,
- die Menge, Art, Grösse und Form von Fremdmaterial im Faserband und deren Verteilung,

Faserparameter:
- die Feinheit und Feinheitsverteilung der das Faserband bildenden Fasern,
- die Länge und die Längenverteilung der das Faserband bildenden Fasern,
- die Oberflächeneigenschaften und Formen der das Faserband bildenden Fasern (Reifegrad),
- Farbe der Fasern

Prozessparameter:
- das Textilmaterial oder die Materialmischung des Faserbandes,
- die Geschwindigkeit des Faserbandes,
- der Spannungszustand des Faserbandes,

Umgebungsparameter:
- die Feuchtigkeit der Fasern und der Umgebung,
- die Temperatur der Fasern und der Umgebung.

Ein Parameter-Set besteht dann im wesentlichen aus den Eigenschaften der Fasern, aus den Prozess-Stufen (Verarbeitungsstufen), aus den Umgebungsbedingungen.

Einige dieser Parameter müssen zeitinvariant gehalten werden, bspw. die Umgebungsparameter, andere werden durch Labormethoden (also statisch) bei verschiedenen Bedingungen ermittelt und als Werte in den Prozess eingebracht bspw. die Stapellänge. Das hier diskutierte Verfahren ist ein dynamisches Messverfahren und wird in seinem Ablauf durch Laborwerte lediglich in der Auswertung unterstützt.

Zwei Faserbänder besitzen die gleiche (dieselbe) Qualität, wenn das ganze Set ihrer Parameterwerte übereinstimmt. Bestimmte Aussagen über den Unterschied zweier Faserbänder in bezug auf einen Parameter können gemacht werden, wenn auf irgend eine Art und Weise alle restlichen Parameterwerte gleichgesetzt werden, oder wenn bekannt ist, ob ein Parameterwert vom anderen abhängig ist, und wenn ja, dann wie. Zu solchen Vergleichszwecken existieren viele standardisierte Laboruntersuchungsmethoden. Für solche Untersuchungen wird bei standardisierten Umgebungsbedingungen und meist mit standardisierten Mengen und standardisierten Anordnungen von Fasern in einem ruhenden Zustand gearbeitet. Auf diese Weise werden von den in der vorhergehenden Aufzählung erwähnten Parametern alle Verarbeitungs-, alle Umgebungsparameter und die meisten Faserbandparameter konstant gehalten, so dass es möglich ist, in befriedigender Weise vor allem Fasern, aber auch Faserbänder und versponnene Produkte zu vergleichen.

Für kontinuierliche Messung der Masse oder der Faserdichte von Faserbändern sind folgende Messmethoden bekannt:

Das Faserband wird, wie in den Europäischen Patentschriften 0'078'393 oder 0'192'835 beschrieben, zur Messung der Masse mechanisch zwischen zwei Walzen komprimiert. Die eine der Walzen ist angetrieben und ortsfest, während die andere geschleppt ist und parallel zu ihrer eigenen Achse verschiebbar. Die verschiebbare Walze übt eine konstante Kraft aus auf das Faserband und wird entsprechend der Komprimierbarkeit des Faserbandes aus ihrer Ruhelage verschoben. Gemessen wird die Auslenkung der geschleppten Walze. Diese Auslenkung ist hauptsächlich vom Fasermaterial abhängig und ist eine Funktion der Fasersubstanzmenge.

Das Faserband wird, wie in der DE-OS 2'323'729 oder in der CH-PS 629' 546 beschrieben, zur Messung der Faserdichte (Titer) in einem Trichter komprimiert und der Staudruck, der durch die aus dem Faserband ausgequetschte Luft entsteht, wird gemessen. Eine Variante dieser Messmethode besteht darin, dass durch das Faserband senkrecht zu seiner Bewegungsrichtung ein Luftstrom von konstantem Druck und konstanter Luftmenge geblasen und der Druckabfall über das Faserband gemessen wird. In beiden Fällen wird eigentlich der ärodynamische Widerstand, den das Faserband einem Luftstrom entgegensetzt gemessen. Eine solche Messung ist hauptsächlich abhängig von der Materialmenge, von der Faserfeinheit, von der Faserorientierung im Faserband, von der Oberfläche und Form der Fasern und von der Geschwindigkeit des Faserbandes.

Messmethoden für Faserbänder, die sich für kontinuierliche Untersuchungen eignen, sind abhängig von mehreren Parametern. Es ist aber für diese Messmethoden, im Gegensatz zu den nicht kontinuierlich arbeitenden Labormethoden, nicht möglich, den Einfluss von mehreren Parametern durch Standardbedingungen (normierte Vorgaben von Bedingungen und/- oder Zeitinvarianz einzelner Bedingungen) auszuschalten. Über den zeitlichen Verlauf und an den verschiedenen Stellen des Herstellungsprozesses des Faserbandes in Öffnerei und Spinnereivorwerk variieren nicht nur die Umgebungsbedingungen und die Verarbeitungsparameter, sondern auch gleichzeitig die Faserband- und die Faserparameter. Da aber die Resultate der kontinuierlichen Überwachung der Faserbandeigenschaften nur für sinnvolle Korrekturmassnahmen und Alarme benutzt werden können, wenn sie auf einzelne Parameter reduziert werden können, ist es wichtig, Mittel und Wege zu finden, um die Messresultate in einzelne, voneinander unabhängige Parameter aufzulösen. Da kurzwellige und langwellige Schwankungen in den Eigenschaften von Faserbändern und Fasern zu verschiedenen Korrektur- oder Alarmreaktionen führen können, ist es empfehlenswert, dass diese beiden Typen von Schwankungen separiert werden.

Wie wichtig eine solche Separierung sein kann, kann an Beispielen gezeigt werden. Fast alle kontinuierlich gewonnenen Messresultate sind abhängig von der Feuchtigkeit des Fasermaterials. Diese Resultate können nicht mit Sollwerten verglichen werden, wenn sie nicht vorgängig auf Standardbedingungen reduziert worden sind. Zur Regulierung der Flockenspeisung und von Streckprozessen sind Daten über die Materialmenge im Faserband, das durch Speisung und Streckung hergestellt wird, notwendig, und zwar unabhängig von den Faserparametern. Für die Regulierung der Ballenöffnung und der Mischer sind Daten notwendig, die die Fasern betreffen, und zwar unabhängig davon, wie viel Material das Faserband, an dem die Messung durchgeführt wird, zum Zeitpunkt der Messung umfasst und wie stark der Parallelisierungsgrad bereits fortgeschritten ist.

Es ist Aufgabe der Erfindung, ein Verfahren aufzuzeigen, mit dessen Hilfe Messungen, die an verschiedenen Orten in Öffnerei und Spinnereivorwerk mit verschiedenen Messmethoden an Faserbändern in verschiedenen Verarbeitungsstufen gemacht werden, so aufgearbeitet werden können, dass daraus auf Standardbedingungen reduzierte, voneinander unabhängige Eigenschaftsparameter oder Schwankungen von Eigenschaftsparametern für die Faserbänder und die in den Faserbändern enthaltenen Fasern erhalten werden können, die direkt miteinander oder mit Sollwerten verglichen und zu Kontroll- und Steuerzwecken benützt werden können. Das Verfahren soll auch die Möglichkeit bieten, langwellige und kurzwellige Schwankungen zu separieren und damit Daten für verschiedene Steuerzwecke bereitzustellen. Ferner ist es die Aufgabe der Erfindung, als spezielle Anwendung des Verfahrens, eine Vorrichtung zu schaffen, mit deren Hilfe zwei Messungen an einem Faserband verarbeitet werden können in eine Angabe über die Materialmenge pro Längeneinheit des Bandes und über Schwankungen in der Faserfeinheit der Fasern, die in dem Faserband enthalten sind. Die beiden Resultate sind unabhängig von der Geschwindigkeit des Faserbandes und auf Standardumgebungsbedingungen reduziert.

Diese Aufgabe wird durch das im kennzeichnenden Teil des unabhängigen Verfahrensanspruches genannte Verfahren und die im kennzeichnenden Teil des unabhängigen Vorrichtungsanspruches genannte Vorrichtung gelöst.

Ein erfindungsgemässes Vorgehen und ein Ausführungsbeispiel einer Messanordnung werden anschliessen mit Hilfe der untenstehenden Figuren diskutiert.

Figur 1    zeigt ein allgemeines Schema des Ablaufes des erfindungsgemässen Verfahrens,

Figur 2    zeigt das Verfahrensschema für eine spezifische Anwendung

Figur 3A    und Figur 3B zeigen eine schematische Darstellung einer Ausführungsvariante der erfindungsgemässen Vorrichtung zur Durchführung des in Figur 2 dargestellten Verfahrens.

Figur 4    zeigt in einer Grafik die Ueberlegung zu den Bedingungen, die an beobachtbare Parameter und Messgrössen gestellt werden.

Figur 5    zeigt in einer schematischen Darstellung die Korrelation der Bandeigenschaften bei von einander beabstandeten Sensoren.

Es geht beim hier diskutierten Verfahren darum, Eigenschaftsparameter des Faserbandes zu messen und aus dem Messresultat zu gewinnen. Es wird davon ausgegangen, dass die Parameter beobachtbar sind, also allgemein gesagt, x (der Messwert) ist eine Funktion von p (die Parameter). Falls eine Umkehrfunktion existiert, gilt auch $p = f^{-1}(x)$. Somit kann man schreiben $x = A \cdot p$, A sind Konstanten in Matrixschreibweise, und falls A invertierbar ist, gilt $p = A^{-1} \cdot x$. Dies ist ein nichtlinearer Zusammenhang, zu bestimmen sind die Elemente von A.

Mit dem Begriff Parameter sind hier im wesentlichen am Band vorhandene Eigenschaften, die vom Ort, das heisst örtlich in Längsrichtung auf dem Band abhängig sind (also nicht zeitlich) und bei bewegtem Band über die Geschwindigkeit mit der Zeit verknüpft sind und dann als Funktion in Abhängigkeit der Zeit behandelt werden können.

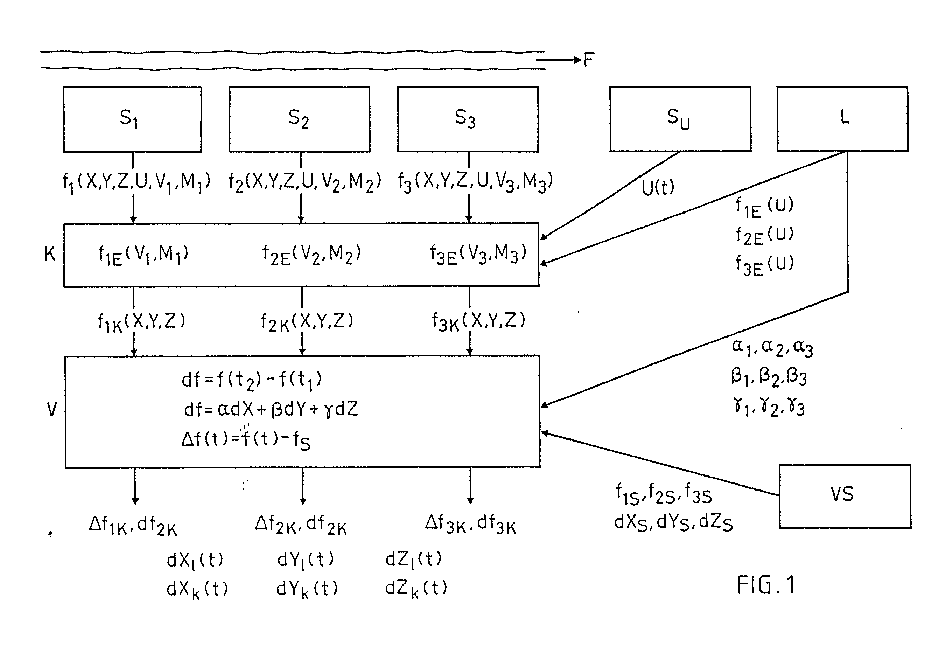

Figur 1 zeigt eine schematische Darstellung des Verfahrensablaufes. Das Faserband läuft schematisch oben von links nach rechts und wird von verschiedenen Sensoren $S_1, S_2$, $S_3$ u.s.w. "abgetastet". Die Sensoren erzeugen Messsignale $f_1$, $f_2$, $f_3$ u.s.w., die abhängig sind von verschiedenen Eigenschaftsparametern X, Y, Z u.s.w. von Faserband und Fasern. Die Anzahl der Sensoren ist beliebig, die Abhängigkeit ihrer Messsignale von den Eigenschaftsparametern verschieden. Ferner sind die Messsignale der Sensoren abhängig von den Umgebungsparametern U, von den Verarbeitungsparametern V und von der spezifischen Messmethode des Sensors, also von Parametern $M_1$ $M_2$, $M_3$. Die Parameter X, Y, Z und U sind ihrerseits Funktionen der Zeit t, während die Verarbeitungsparameter V vom Messort abhängig sind.

Die Messsignale $f_1$, $f_2$ $f_3$, u.s.w. werden alle in eine Kalibriereinheit K geführt. Die Kalibriereinheit erhält ferner von Sensoren $S_u$ die von der Zeit abhängigen Werte für die Umgebungsparameter U, wie zum Beispiel die Werte für die Luftfeuchtigkeit und für die Temperatur. Von einer Stelle, an der statische Messungen bei standardisierten Bedingungen mit Labormethoden durchgeführt werden, erhält die Kalibriereinheit Eichfunktionen oder Eichmatrizen $f_{1E}(U)$, $f_{2E}(U)$, $f_{3E}(U)$, u.s.w. für die Abhängigkeit der Messwerte von den Umgebungsbedingungen U für das im Moment verarbeitete Fasermaterial oder Fasermaterialmischung. Die Kalibriereinheit K besitzt ihrerseits Eichfunktionen $f_{1E}(M_1,V_1)$, $f_{2E}(M_2,V_2)$, $f_{3E}(M_3,V_3)$, u.s.w. oder entsprechende mathematische Algorithmen, das heisst Kenntnis über die Abhängigkeit der Messfunktionen von der Messmethode und von den Verarbeitungsparametern. Mit Hilfe aller Eichfunktionen ermittelt die Kalibriereinheit kalibrierte Messfunktionen $f_{1K}(X,Y,Z)$, $f_{2K}(X,Y,Z)$, $f_{3K}(X,Y,Z)$, u.s.w., die nur noch von den Eigenschaftsparametern X, Y, Z, u.s.w. abhängig sind, die ihrerseits Funktionen der Zeit sind. In bezug auf Umgebungsbedingungen und Verarbeitungsbedingungen sind sie auf Standardbedingungen reduziert. Eine typische Funktion der Kalibriereinheit K ist zum Beispiel die Elimination der Abhängigkeit von der Bandgeschwindigkeit, mit der pneumatische Messmethoden behaftet sind, indem das Messsignal über eine variable Zeit, die einer konstanten Bandlänge entspricht, integriert wird.

Die kalibrierten Messfunktionen $f_{1K}$, $f_{2K}$, $f_{3K}$, u.s.w. werden in eine Verarbeitungseinheit geführt. Die Verarbeitungseinheit besitzt mathematische Algorithmen, die es ihr erlauben, die kalibrierten Messfunktionen so zu kombinieren, dass als Resultat voneinander unabhängige Eigenschaftsparameter resultieren, die nur noch Funktionen der Zeit sind. Es sind hier die verschiedensten Varianten denkbar, von komplexen Eichfunktionen bis zu komplexen Gleichungssystemen etc.. Aufgabe der Verarbeitungseinheit ist es, voneinander unabhängige Werte für die Eigenschaftsparameter zu produzieren, also die eigentlichen Funktionen X(t), Y(t), Z(t), u.s.w. oder für andere Anwendungsfälle die zeitlichen Veränderungen dieser Funktionen dX(t), dY(t), dZ(t), u.s.w. Diese Werte werden dann für Steuerungs- und Kontrollzwecke weiterverwendet.

Als Beispiel für eine Ausführungsform der Verarbeitungseinheit soll im Folgenden der Fall aufgezeichnet werden, der sich an die obige Beschreibung für den Fall mit drei Sensoren und drei Eigenschaftsparametern mühelos anschliesst, nämlich der Fall, in dem die Absolutwerte der kalibrierten Messfunktionen und die zeitlichen Veränderungen der einzelnen Eigenschaftsparameter genügend Informationen liefern. Für diesen Fall genügt der einfache Algorithmus der als Matrix A darstellbaren linearen Kombination. Es kann in erster Näherung angenommen werden, dass die einzelnen Eigenschaftsparameter X, Y, Z u.s.w. voneinander unabhängig sind und dass ihr Änderungsbereich klein ist. Die Veränderung der Messfunktion über eine kleine Zeitspanne kann dann aufgefasst werden als die Summe der Veränderungen aller sie bestimmenden, ihrerseits von der Zeit abhängigen Parameter. Es gilt also in erster Näherung für jede kalibrierte Messfunktion:

$$d f_k = \frac{\partial f_k}{\partial X} dX + \frac{\partial f_k}{\partial Y} dY - \frac{\partial f_k}{\partial Z} dZ$$

Wenn die einzelnen Ableitungen der Parameter nach der Zeit näherungsweise als konstant betrachtet werden, dann gilt:

$$d f_k = \alpha \, dX + \beta \, dY - \gamma \, dZ$$

Die Faktoren $\alpha$, $\beta$ und $\tau$ müssen unter stationären, standardisierten Bedingungen im Labor mit dem zu verarbeitenden Fasermaterial ermittelt werden. Für drei Sensoren, die von drei Eigenschaftsparametern abhängig sind, ergibt sich dann ein lineares Gleichungssystem mit drei Unbekannten, das von der Verarbeitungseinheit auf bekannte Weise aufgelöst werden kann:

$$df_{1k} = \alpha_1 dX + \beta_1 dY + \gamma_1 dZ$$
$$df_{2k} = \alpha_2 dY + \beta_2 dY + \gamma_2 dZ$$
$$df_{3k} = \alpha_3 dX + \beta_3 dY - \gamma_3 dZ$$

Dabei ist zu beachten, dass jede dieser Gleichungen entweder für ein bestimmtes Zeitintervall oder für einen bestimmten Zeitpunkt gilt. Liegendie Sensoren nahe beieinander kann näherungsweise dasselbe Zeitintervall bzw. derselbe Zeitpunkt für alle Sensoren genommen werden, liegen die Sensoren weit auseinander, müssen die Zeitintervalle bzw. Zeitpunkte in Abhängigkeit von der Bandgeschwindigkeit so gewählt werden, dass sie dem Durchgang desselben Bandabschnittes entsprechen. Das Auflösungsvermögen bleibt in jedem Falle dasselbe, abhängig vor allem von der Länge des Zeitintervalles, während aber der Zeitraum zwischen der ersten Messung und dem Vorliegen des Messresultates für weit auseinanderliegende Messstellen grösser wird.

Als Beispiel für Sensoren, die "weit" auseinanderliegen sei folgende Messanordnung genannt: $F_B$ wird an einer Stufenwalze beim Kardenauslauf gemessen und $F_B + F_F$ mit einem aktiv-pneumatischen Messtrichter am Bandeinlauf in der Bandablage gemessen. Auf diese Weise können die Sensoren bis einige Meter voneinander beabstandet sein. Bei solch einer Anordnung weist die Bandablage natürlich einen autonomen Antrieb auf.

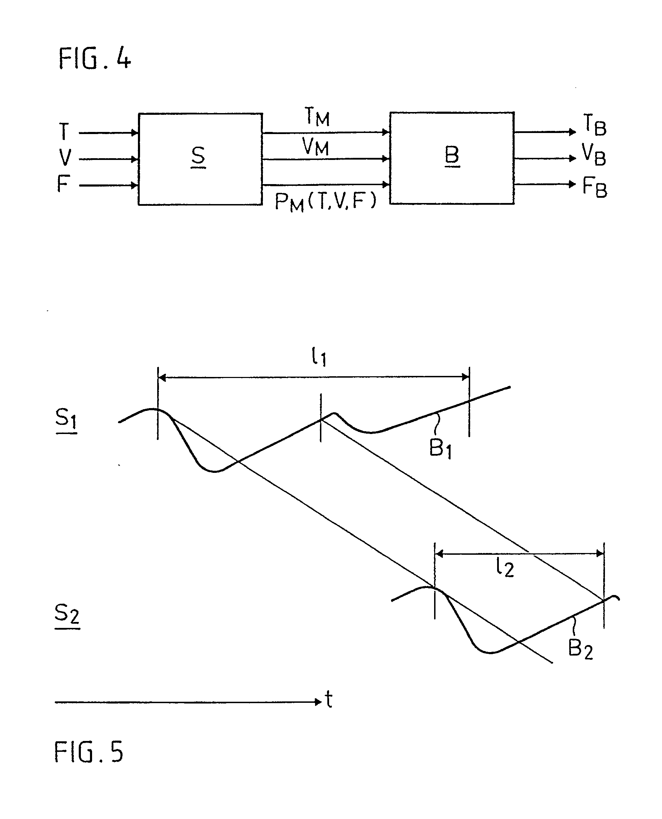

Bei der Messung mit weitauseinanderliegenden Sensoren muss die Korrelation zwischen den beiden Messungen gesichert sein, das heisst, das Band muss trotz weit auseinander liegenden Messstellen am gleichen Bandabschnitt gemessen werden, was durch eine Korrelation (siehe Figur 5) bewerkstelligt wird. Auf diese Weise wir ein Ausmessen der Wegstrecke, die das Band zwischen den beiden Messstellen zurücklegen muss, umgangen und die Freiheit bezüglich Ort der Messung und Reihenfolge der Messung kann voll genützt werden, was sich im Online-Betrieb natürlich sehr vorteilhaft bemerkbar macht. Der Korrelationsteil wird weiter unten im Zusammenhang mit der Figur 5 noch diskutiert werden.

Zurückkommend auf die obenstehenden Gleichungen: Als absolute Werte fallen nur die Werte der kalibrierten Messfunktionen $f_{1K}(t)$, $f_{2K}(t)$, $f_{3K}(t)$, u.s.w. an. Für Steuerungszwecke, die mit absoluten Werten arbeiten, müssen also im vorliegenden Falle Sollwerte $f_{1S}$, $f_{2S}$, $f_{3S}$, u.s.w. vorliegen, die dann in der Verarbeitungseinheit periodisch mit den zeitlichen Werten der kalibrierten Messfunktionen verglichen und zu den entsprechenden Differenzen verarbeitet werden.

Die Veränderungen von Eigenschaftsparametern können in der Verarbeitungseinheit V über kürzere oder längere Zeitintervalle gemittelt werden. Aus diesen Mittelwerten lassen sich dann kurzwellige Schwankungen $dX_k$, $dY_k$' $dZ_k$, u.s.w. und langwellige Schwankungen $dX_1$, $dY_1$, $dZ_1$, u.s.w ermitteln. Für das Steuerungs- oder Alarmsystem können diese Schwankungen aufsummiert werden und dauernd mit von der Verfahrenssteuerung VS vorgegebenen Grenzwerten $dX_S$, $dY_S$, $dZ_S$, u.s.w. verglichen werden.

Ausführungsvarianten des beschriebenen Verfahrens bestehen darin, dass die Sensoranordnung bezüglich Reihenfolge und bezüglich der gegenseitigen Distanz beliebig ist. Gemäss Figur 1 lassen sich $S_1$, $S_2$, $S_3$ (für $S_3$ hier die Messung der Faserband-Geschwindigkeit) und so fort beliebig permutieren, was zur Realisierung von Online-Lösungen in Prozessanlagen ein unschätzbarer Vorteil ist. Ferner bestehen Aussführungsvarianten des Verfahrens darin, dass die Kalibrierung erst nach der Verarbeitung erfolgt oder dass die Kalibrierung oder Teile davon in die Verarbeitung integriert werden. Dies kann man sich so vorstellen, dass zum Beispiel der Einfluss der Feuchtigkeit auf die Messresultate nicht durch eine Reduktion der Messresultate auf Standardbedingungen, sondern durch einen weiteren Term in der Gleichung der linearen Kombination der Verarbeitung, einen sogenannten Störterm, einbezieht. In diesen Störterm können auch noch andere Einflüsse miteinbezogen werden. Für die Bestimmung des Störtermes sind aber genau wie für eine Kalibrierung Eichmessungen notwendig.

Das beschriebene Verfahren ist ein sehr allgemeines Ausführungsbeispiel des erfindungsgemässen Verfahrens. Es liefert mit drei oder mehr Sensoren, deren Messfunktionen von denselben drei oder mehr Eigenschaftsparametern abhängig sind die zeitlichen Veränderungen der drei Eigenschaftsparameter und die absoluten kalibrierten Werte der Messfunktionen. Von den einzelnen Eigenschaftsparametern kann es keine Absolutwerte liefern. Soll dies der Fall sein, müssen einzelne der Sensoren spezifisch sein, das heisst, dass ihre Messfunktion nach der Kalibrierung nur noch von einem Eigenschaftsparameter abhängt. Für wichtige Steuerungsparameter, wie es zum Beispiel die Materialmenge pro Bandlänge ist, ist es vorteilhaft, spezifische Sensoren, das sind Sensoren, die den zu messenden physikalischen Wert direkt proportional anzeigen können und nicht auf indirektem Weg ermittelt werden muss, einzusetzen, während die Änderungen von weniger wichtigen Parametern von Sensorkombinationen (also auf indirektem Weg) ermittelt werden können. Da es aber nicht für jeden Eigenschaftsparameter einen spezifischen Sensor gibt, ist die Sensorkombination in gewissen Fällen die einzige Möglichkeit Eigenschaftsparameter in ihrem zeitlichen Verlauf zu verfolgen.

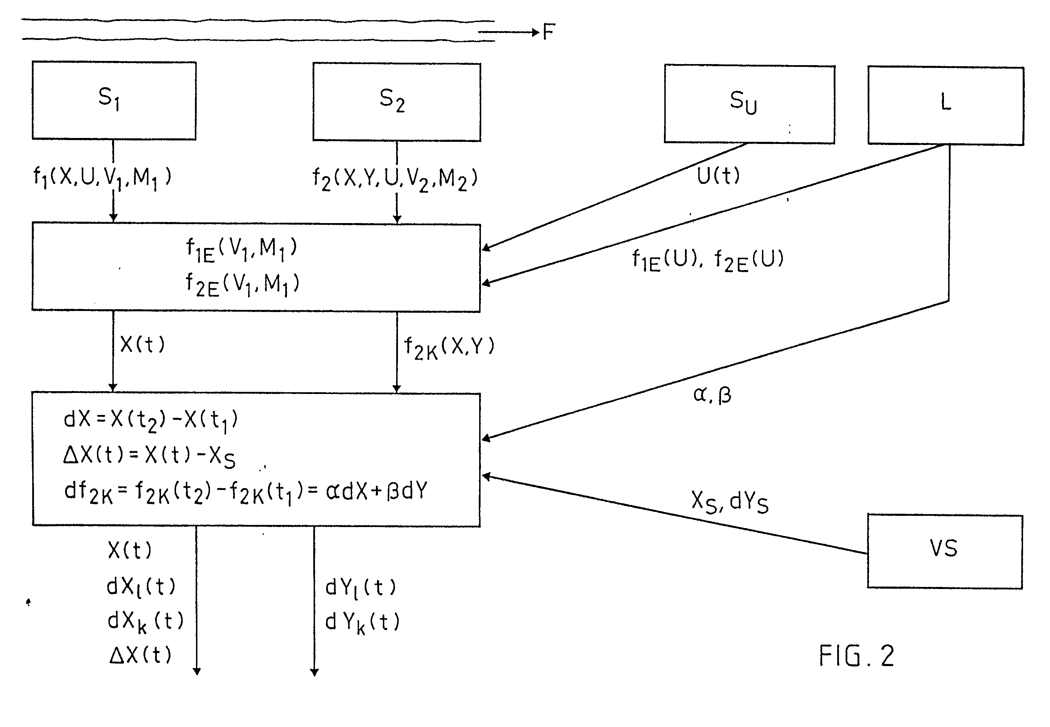

Als weiteres Anwendungsbeispiel des erfindungsgemässen Verfahrens, auf das sich dann auch die erfindungsgemässe Vorrichtung bezieht, soll sich denn auch auf eine Kombination eines spezifischen Sensors mit einem nicht spezifischen Sensor aufbauen. Der schematische Verfahrensablauf ist aus der Figur 2 ersichtlich. Sensor $S_1$ ist ein spezifischer Sensor für den Eigenschaftsparameter X. Er liefert eine Messfunktion, die von dem einen Eigenschaftsparameter X und von den Umgebungs-, Verarbeitungs- und Messparametern U, $V_1$, und $M_1$ abhängig ist. Der Sensor $S_2$ ist ein nicht spezifischer Sensor, der von den beiden Eigenschaftsparametern X und Y abhängt. Die Kalibriereinheit arbeitet in der für den allgemeinen Fall beschriebenen Weise und liefert die beiden kalibrierten Messfunktionen $f_{1K}(X) = X(t)$ und $f_{2K}(X,Y)$. Die Verarbeitungseinheit V braucht lediglich die eine Gleichung aufzulösen und an ihrem Ausgang sind die Funktionen X(t), dX(t) und dY(t) sowie deren Abweichungen von den entsprechenden vorgegebenen Werten der Verfahrenssteuerung erhältlich. Auch hier ist die Vertauschbarkeit der Messfolge gegeben, zeitlich und räumlich, und die Distanz zwischen den beiden Sensoren unerheblich, da der zeitliche Ablauf der Messung durch Korrelation verknüpft ist. Wie diese Verknüpfung stattfindet, wird im Zusammenhang mit Figur 5 diskutiert. Für die Messung und den Umgang mit dem Parameter V (Geschwindigkeit), sei auf die CH-PS 629'546 verwiesen.

Ein konkreter Anwendungsfall für das in Figur 2 dargestellte Verfahren ergibt sich für den Fall der Kombination der beiden Eigenschaftsparameter Materialmenge pro Bandlänge und Faserfeinheit. Für die Materialmenge pro Bandlänge bieten sich verschiedene Sensoren als spezielle Sensoren an, wenn einige Vernachlässigungen gemacht werden dürfen. Für die Faserfeinheit besteht nach dem Stand der Technik kein spezifischer Sensor, dagegen können sich einige Sensoren, wieder mit einigen Vernachlässigungen, als kombinierte Sensoren für Materialmenge pro Bandlänge und für die Faserfeinheit verwenden lassen. Für den speziellen Anwendungsfall wäre also die Materialmenge pro Bandlänge gleich dem Eigenschaftsparameter X der Figur 2, während die Faserfeinheit dem Eigenschaftsparameter Y entspräche.

Als Sensor zur Messung der Materialmenge pro Bandlänge $S_1$ kann ein bspw. ein mechanischer Sensor (Walzenpaar), verwendet werden. Als Sensor $S_2$ für die Messung der Materialmenge zusammen mit der Faserfeinheitkann ein pneumatischer Sensor verwendet werden. Die beiden Sensoren sind so voneinander entfernt, wie es durch die Prozess-Anlage örtlich gegeben ist, was auch für die Reihenfolge der Messstellen gilt, wobei sie durch Korrelation miteinander verbunden sind. Kann man annehmen, dass sich die Eigenschaftsparameter von einem Sensor zum anderen zwischenzeitlich nicht verändern und dass für die Bestimmung der zeitlichen Veränderung der Faserfeinheit dasselbe Zeitintervall für beide Messungen genommen werden kann, was bei kleiner Wegdistanz zwischen den Sensoren und bei hoher Bandgeschwindigkeit der Fall sein kann, dann kann die Korrelation auf Null gesetzt, also auf sie verzichtet werden. Die Kalibriereinheit arbeitet wie beschrieben, wobei für die Kalibrierung bezüglich der Bandgeschwindigkeit die Methode der Integration über konstante Bandlängen verwendet wird. Von den an der Verarbeitungseinheit erhältlichen Daten sind besonders die langwelligen Abweichungen der Materialmenge pro Bandlänge für die Steuerung der Speisung und eventuell der Streckprozesse und die langwelligen Schwankungen der Faserfeinheit für die Überwachung der Öffnerei wichtig.

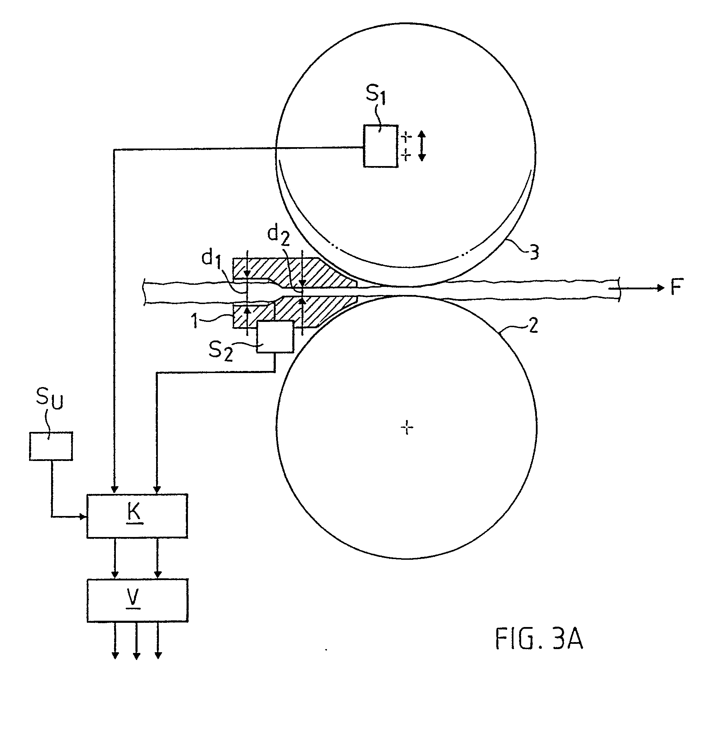

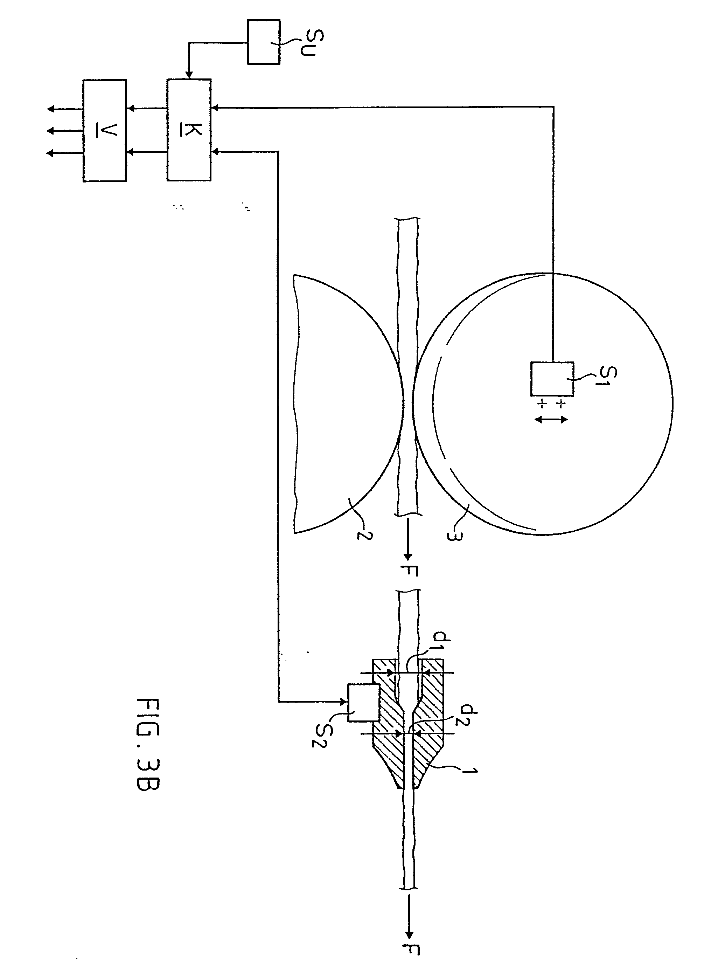

Figur 3A zeigt schematisch ein Ausführungsbeispiel einer erfindungsgemässen Vorrichtung zur Ausführung des speziellen, in Figur 2 schematisch dargestellten Verfahrens zur Bestimmung der Materialmenge pro Bandlänge und den Schwankungen in der Faserfeinheit. Die Messstellen (Sensoren) können zum Beispiel Einlauf oder Auslauf einer Strecke oder am Auslauf der Karde oder an der Bandablage installiert sein. Hier sind sie relativ nahe beieinander liegend in der Reihenfolge: von der Sensorkombination abhängiger Sensor (Bandtrichter) und spezifischer Sensor (Stufenwalze). In Figur 3B sind die beiden Sensoren vertauscht und "weit voneinander liegend".

Das Faserband F durchläuft einen Trichter 1, in dem es von einem Durchmesser d1 auf einen Durchmesser d2 komprimiert wird. An der Verengungsstelle wird von einem entsprechenden Sensor $S_2$ der durch die Komprimierung entstehende Staudruck gemessen. Der Trichter 1 mündet in den Spalt zwischen zwei Walzen 2 und 3. Die Walze 2 ist ortsfest und angetrieben. Die Walze 3 wird geschleppt und ist parallel zu ihrer Längsachse auf der Ebene der beiden Walzenlängsachsen verschiebbar. In der Ruhestellung der Walze 3 ist der Abstand zwischen den beiden Walzen an seiner engsten Stelle etwas kleiner als der Durchmesser der Austrittsöffnung des Messtrichters 1. Das Faserband wird also zwischen den beiden Walzen in jedem Falle komprimiert. Da das Faserband der Komprimierung Widerstand entgegensetzt, wird die Walze 3 verschoben. Ein Sensor $S_1$ misst die Auslenkung der Walze 3.

Die beiden Messfunktionen werden in der Kalibriereinheit K auf Standardumgebungsbedingungen reduziert und die pneumatische Messung wird durch Integration über eine konstante Bandlänge von ihrer Abhängigkeit von der Bandgeschwindigkeit befreit. Die Kalibriereinheit ist auch dafür verantwortlich, mit Hilfe entsprechender Eichwerte den unterschiedlichen Spannungszustand des Faserbandes vor und nach der Komprimierung im Trichter in die Messresultate einzubeziehen, wenn dieser Einfluss nicht als vernachlässigbar betrachtet wird. Die Verarbeitungseinheit V verarbeitet, wie in der Beschreibung der Figur 2 beschrieben, die kalibrierten Messfunktionen. An ihrem Ausgang sind der Absolutwert der Materialmenge pro Bandlänge, sowie ihre zeitlichen Schwankungen und die zeitlichen Schwankungen der Faserfeinheit erhältlich. Wenn die Verarbeitungseinheit entsprechende Sollwerte erhält, sind auch die Abweichungen der Grössen von diesen erhältlich. Die Sollwertabweichungen der Materialmenge pro Bandlänge wird weiterverwendet, um vorgehende Speisungsvorrichtungen und nachfolgende Streckprozesse zu steuern. Die Abweichungen von der vorgegebenen Faserfeinheit werden zur Korrektur der Mischung oder für Alarmmeldungen bei unzulässigen Schwankungen verwendet.

Figur 3B zeigt nun eine Ausführungsvariante, bei welcher der Bandtrichter beabstandet vom spezifischen Sensor, das ist hier ein Stufenrad, angeordnet ist, bspw. nahe bei der Bandablage. Der Bandtrichter ist ein Sensor, der sich an vielen Stellen anbringen lässt und man sollte diese Möglichkeiten auch nützen. Zwischen dem Sensor 1 und dem Sensor 2 verläuft das Band, dessen Laufdistanz durch eine Korrelationsrechnung erfasst wird, das heisst, dass gegebenenfalls der Sensor 2 mitten im Prozessablauf in eine andere Position gebracht werden kann, ohne dass die Faserfeinheitsmessung beeinträchtigt wird. Diese Flexibilität kommt der Automatisierung der Gesamtanlage zu statten.

Die Figuren 4 und 5 zeigen schematisch Verfahrensdetails, die hier separat noch diskutiert werden. Figur 4 zeigt den Messablauf vom Standpunkt des Beobachters aus gesehen. Es wurde eingangs schon festgehalten, dass die Parameter beobachtbar sein müssen; der Beobachter ist hier der Teil des Verfahrens, der die Parameter gewissermassen "sichtbar" macht. Zwei Blackboxes illustrieren diesen Sachverhalt, sie sind miteinander durch die Mess-Signale verbunden. In die Blackbox S (für Sensorik) fliessen physikalische Grössen (Fadentiter T, Geschwindigkeit V, Faserfeinheit F usw.) und erscheinen am Ausgang als Mess-Signale $T_M$, als Auslenkung der Stufenwalze, $V_M$, als Puls/Zeiteinheit des Tachos, $P_M(T,V,F_{FA-SERBAND})$ als Druckdifferenz des Druckmessers am Bandtrichter. Diese Mess-Signale werden der Blackbox B (für Beobachter) zugeführt, in welcher sie zu Messwerten der physikalischen Eingangsgrössen in die Blackbox S umgewertet werden und als $T_B$, für den Titer, als $V_B$, für die Geschwindigkeit, als $F_B$, für die Faserfeinheit, usw. erscheinen. Man sieht, dass in diesem Beispiel für die physikalische Grösse F, die als Parameter F-$(T,V,F_{FASERBAND})$ ungetrennt von weiteren Parametern in das Messverfahren einläuft, diesen als F getrennt von anderen Parametern wieder verlässt.

Figur 5 zeigt schematisch die bereits erwähnte Korrelation, die es ermöglicht die Messungen von zwei voneinander entfernten Sensoren in der erfindungsgemässen Art und Weise zu kombinieren. Die Eigenschaften des Faserbandes, die in erster Näherung als zeitunabhängig angenommen werden, werden bei laufendem Faserband von jedem Sensor als Funktionen der Zeit registriert. Dabei entspricht jeder Zeitabschnitt einem Bandabschnitt einer bestimmten Länge. Derselbe Bandabschnitt durchläuft den Messbereich von zwei voneinander entfernten Sensoren zu verschiedenen Zeiten. Die Zeitdifferenz, die verstreicht bis ein Bandabschnitt von einem Sensor $S_1$ zu einem folgenden Sensor $S_2$ gelangt, ist abhängig von der Bandgeschwindigkeit und von der Distanz zwischen den beiden Sensoren. Sowohl die Bandgeschwindigkeit als auch die Distanz zwischen den Sensoren ist grössenordnungsmässig bekannt, kann aber fein varieren (beispielsweise durch den Durchhang des Bandes zwischen den Sensoren). Eine Korrelationsrechnung erlaubt es nun, die Messungen der beiden Sensoren trotzdem genau zu synchronisieren. Die beiden Sensoren $S_1$ und $S_2$ messen bestimmte Eigenschaftsparameter des Faserbandes als Funktion der Zeit. Die Messwerte werden über entsprechende Beobachter einem Korrelationssystem zugeführt, wo sie in "Eigenschaftsabbildungen" von Bandabschnitten konstanter Länge $l_1$ oder $l_2$ umgewandelt werden. Die "Eigenschaftsabbildungen" $B_1$ des in Transportrichtung des Faserbandes ersten Sensors $S_1$ werden gespeichert, diejenigen ($B_2$) des zweiten Sensor $S_2$ mit den gespeicherten verglichen. Dieser Vergleich kann mit einer Korrelationsrechnung durchgeführt werden, wobei eine Schwelle für die minimal geforderte Korrelationsgüte vorgegeben wird. Zeitabschnitte, für die die "Eigenschaftsabbildungen" übereinstimmen, entsprechen gleichen Bandabschnitten und der Verlauf der Faserbandeigenschaften in diesen Abschnitten kann der erfindungsgemässen kombinierten Auswertung zugeführt werden. Für die beiden Sensoren, die für die kontinuierliche Bestimmung der Faserfeinheit von Fasern in Bändern notwendig sind, ist eine solche Korrelation dann erfolgreich, wenn der Einfluss der Faserfeinheit, der ja einen Unterschied zwischen den zwei Messungen bedingt, für die Korrelation, die eigentlich gleiche Messungen voraussetzt, vernachlässigbar ist, was in erster Näherung für die üblicherweise verarbeiteten Faserbänder angenommen werden darf. Eine weitere Bedingung für die Anwendbarkeit der Korrelation im oben genannten Sinne ist, dass zwischen den beiden Sensoren das Faserband keiner Veränderung (z.B. Strecken) unterzogen werden darf.

In der Korrelationsrechnung wird ein Mass für die Aehnlichkeit zweier Signale berechnet. Ein zu verwendender Korrelationsalgorithmus wird durch entsprechende Vorgaben und Rahmenbedingungen so einfokussiert, dass nur noch die Erwartungswerte überprüft werden müssen. Dazu ist nochmals Figur 4 herangezogen. Der Beobachter "sieht" an den Sensoren Signale, die vom Titer oder von der Geschwindigkeit oder von der Fasermasse im Band determiniert sind, und extrahiert aus diesen Signalen Eigenschaften des Faserbandes gemäss einem vorgegebenen Modell. Dieses Modell wird nun um einen Korrelationsalgorithmus erweitert, mit dem die Aehnlichkeit zweier Signale, nämlich ein Signal eine gemäss Prozessablauf stromaufwärts messenden Sensor zu einem bestimmten Zeitpunkt und eines stromabwärts messenden Sensors zu einem späteren Zeitpunkt. Die messenden Sensoren müssen dabei nicht von der gleichen Gattung sein, denn der Beobachter von Figur 4 sorgt dafür, dass die physikalischen Grössen zum Vergleich kommen. Wird beispielsweise der Fadentiter T mit dem stromaufwärts liegenden Sensor gemessen und eine Funktion f(T..) von einem Sensor der später misst ermittelt, so sind beide Messungen durch T determiniert und können so verglichen werden. Es ist dann unerheblich, wenn das "Messresultat" dem Prozessablauf nachhinkt, das heisst, wenn nachträglich die gewünschte Korrelation festgestellt wird. Sie bildet dann die Basis für einen neuen Erwartungswert.

So wird kontinuierlich über einen Zeitabschnitt $l_1$ am Sensor $S_1$ eine Eigenschaftsabbildung $B_1$ aufeinanderfolgendert Fasertiterwerte kollektioniert und gleichzeitig am Sensor $S_2$ aus f(T,V,F) die Fasertiterwerte extrahiert und korreliert. Die als Masszahl definierte mittlere quadratische Abweichung zeigt Abweichungen überproportional stark an, sodass sie zur Ermittlung der Signaldifferenzen $\delta(t)$ recht gut zu handhaben ist. Ein daraus abgeleitetes normiertes Abweichungsmass kann dann als Aehnlichkeitsmass für die aus T determinierten Signale verwendet werden. Der entsprechende Korrelationskoeffizient ist eine Zahl zwischen -1 und +1, wobei +1 die grösste Aehnlichkeit bezeichnet. Für eine feste zeitliche Lage der verglichenen Signale zueinander gilt der Korrelationskoeffizient als Mass. Für die zeitliche Verschiebung zwischen den beiden Signalen gilt die Korrelationsfunktion bzw. die normierte Autokorrelationsfunktion. Ein definierter Erwartungswert bestimmt die Anzahl von Proben und eine Vertrauenschwelle bestimmt die Güte der Korrelation. Simultan dazu wird aus der Funktion f(T,V,F) die Faserfeinheit extrahiert, deren Verwertung von der Güte der Korrelation abhängig gemacht werden kann.

Die den korrelierten Eigenschaftsabbildungen B1,B2 zugehörige Zeitgrössen, das ist der zeitliche Abstand zwischen zwei einander zugeordneten Eigenschaftsabbildungen, können zur Bildung von Erwartungswerten verwendet werden. Auf diese Weise kann für den Messvorgang stromabwärts eine Stelle mit gemessenen Eigenschaften im Faserband (auch zeitlich) erwartet werden, sodass sich die Messung der zweiten Eigenschaftsabbildung und die Korrelation sukzessive erübrigt und nur noch stichprobenartig durchgeführt wird.

Man muss sich im klaren sein, dass die beschriebene Vorrichtung nicht imstande ist, den Parameter Faserfeinheit von den Eigenschaftsparametern Oberflächenbeschaffenheit und Form der Fasern zu trennen und dass sie Schwankungen im Fasermaterial oder Materialmischung, in der Verschmutzung des Faserbandes oder in der Orientierung der Fasern im Faserband als Schwankungen in der Materialmenge pro Bandlänge interpretieren wird.

## EP 0 431 481 A1

**Ansprüche**

1. Verfahren zur kontinuierlichen Ermittlung der Faserfeinheit und Schwankungen der Faserfeinheit in Faserbändern, dadurch gekennzeichnet, dass die Daten aus Signalen mindestens zweier Sensoren verschiedener Gattung $(S_1, S_2)$ miteinander verarbeitet werden, wobei aus den Signalen von mindestens einem der Sensoren $(S_1)$ in einer Verarbeitungseinheit (Beobachter) mit Hilfe der Signale eines anderen Sendors $(S_1)$ einzelne, voneinander unabhängige Eigenschaftsparameter oder deren zeitliche Schwankungen ermittelt werden, die im Prozess nicht direkt und spezifisch gemessen werden können.

2. Verfahren nach Anspruch 1, dadurch gekennzeichnet, dass Eigenschaftsparameter aus Daten von Signalen einzelner Sensoren, die mehrere Eigenschaftsparameter enthalten, mit Daten aus Signalen anderer Sensoren, die nur einen Eigenschaftsparameter enthalten, separiert werden.

3. Verfahren nach Anspruch 1, dadurch gekennzeichnet, dass gleichzeitig Sensoren verwendet werden, deren abgegebenen Signale $(P_M)$ Information über mehrere Eigenschaftsparameter (T,F) und Sensoren $(S_1)$ deren abgegebenen Signale $(T_M)$ Information über einen Eigenschaftsparameter (T) enthalten.

4. Verfahren nach einem der Ansprüche 1 bis 3, dadurch gekennzeichnet, dass Sensoren verschiedener Gattung $(S_1, S_2)$ am selben Faserbanddurchgang in beliebiger Reihenfolge und in der gewünschten Distanz zueinander angeordnet werden.

5. Verfahren nach Anspruch 4, dadurch gekennzeichnet, dass zwischen zwei (nicht zwangsweise unmittelbar) aufeinander folgende Sensoren eine Korrelation bestimmter Signale durch Eigenschaftsabbildungen (B1,B2) hergestellt wird.

6. Verfahren nach Anspruch 5, dadurch gekenneichnet, dass die Eigenschaftsabbildung (B1,B2) durch Messungen und Messdaten-Speicherung von Faserbandabschnitten $(l_1, l_2)$ desselben Faserbandes (F) hergestellt wird, deren zeitlicher Abstand (t) voneinander bestimmt wird und dass beim Vergleich von Eigenschaftsabbildungen eines Faserbandabschnittes bei Unterschreiten einer vorgegebenen maximalen Abweichungsgrösse gemessene Faserbandabschnitte einander zugeordnet werden und der zugehörige zeitliche Abstand (t) dazu verwendet wird, Messdaten $(T_M, V_M, P_M)$ von Sensoren verschiedener Gattung $(S_1, S_2)$ einander zuzuordnen.

7. Verfahren nach Anspruch 6, dadurch gekennzeichnet, dass den korrelierten Eigenschaftsabbildungen (B1,B2) zugehörige Zeitgrössen (zeitlicher Abstand zwischen zwei einander zugeordneten Eigenschaftsabbildungen) zur Bildung von Erwartungswerten verwendet werden.

8. Verfahren nach Anspruch 7, dadurch gekennzeichnet, dass die Erwartungswerte zur Messung von Faserbandabschnitten für die Bildung von zeitlich nachfolgenden Eigenschaftsabbildungen zur Korrelation herangezogen werden.

9. Verfahren nach einem der Ansprüche 1 bis 8, dadurch gekennzeichnet, dass die Messdaten zu ihrer Reduktion auf Standardbedingungen in bezug auf Umgebungs- Verarbeitungs- uns Messparameter und einer Verarbeitung zu ihrer Kombination untereinander und mit vorgegebenen Sollwerten kalibriert werden.

10. Verfahren nach Anspruch 9, dadurch gekennzeichnet, dass die Kalibrierung mit entsprechenden Eichfunktionen, die durch kontinuierliche Messverfahren ermittelt werden,bzw. darauf basierenden mathematischen Algorithmen durchgeführt wird.

11. Verfahren nach Anspruch 9, dadurch gekennzeichnet, dass die Verarbeitung mit entsprechenden Eichfunktionen, die durch nicht kontinuierliche Messmethoden ermittelt werden, bzw. darauf basierende mathematischen Algorithmen durchgeführt wird.

12. Verfahren nach einem der Ansprüche 10 oder 11, dadurch gekennzeichnet, dass als mathematischer Algorithmus für die Verarbeitung die lineare Kombination der Ableitungen der Messfunktionen nach den einzelnen Eigenschaftsparametern verwendet wird.

9

13. Verfahren nach Anspruch 12, dadurch gekennzeichnet, dass zwei Messungen durchgeführt werden, wobei die eine nur von der Materialmenge pro Bandlänge abhängig ist, die andere von der Materialmenge pro Bandlänge und von der Faserfeinheit.

14. Vorrichtung zur kontinuierlichen Messung der Faserfeinheit in einem Faserband mit mindestens zwei Sensoren ($S_1$,$S_2$) und einer Verarbeitungseinheit der gewonnenen Signale aus den Sensoren zu Fasereigenschaftsparametern, dadurch gekennzeichnet, dass mindestens ein Sensor ($S_1$) so ausgelegt ist, dass er ein Signal ($T_M$) proportional zu eine Faserbandeigenschaft (T) abgibt, die in einem anderen Sensor ($S_2$) der Vorrichtung zusammen mit anderen Faserbandeigenschaften (T,F) gemessen und als Messsignale anfallen und durch eine Verabeitungseinheit, in welcher aus den Messdaten eine nicht direkt messbare Faserbandeigenschaft ermittelt wird.

15. Vorrichtung nach Anspruch 14, dadurch gekennzeichnet, dass sie einen Sensor ($S_1$) zur Messung der Materialmenge pro Bandlänge und einen Sensor ($S_2$), der die Materialmenge pro Bandlänge sowie die Faserfeinheit misst, aufweist.

16. Vorrichtung nach einem der Ansprüche 14 oder 15, dadurch gekennzeichnet, dass sie eine Kalibriereinheit aufweist.

17. Vorrichtung nach einem der Ansprüche 14 bis 16, dadurch gekennzeichnet, dass der Sensor zur Messung der Materialmenge pro Bandlänge auf einer Messung des mechanischen Widerstandes des Bandes bei Kompression des Faserbandes oder der Veränderungen der Eigenschaften des elektrischen Feldes einer Kondensatoranordnung, durch die das Band läuft, beruht.

18. Verfahren nach einem der Ansprüche 14 bis 16, dadurch gekennzeichnet, dass der Sensor, der die Materialmenge pro Bandlänge zusammen mit der Faserfeinheit misst, auf einer Messung des Staudruk-kes, der bei einer Komprimierung des Bandes entsteht, beruht.

19. Vorrichtung nach den Ansprüchen 17 und 18, dadurch gekennzeichnet, dass der Sensor zur Messung der Materialmenge pro Bandlänge die Auslenkung der einen, beweglichen Walze eines Walzenpaares, zwischen dem das Band hindurchläuft und komprimiert wird, misst, und der während der Sensor der die Materialmenge pro Bandlänge zusammen mit der Faserfeinheit ders Bandes miss, ein pneumatischer Sensor ist, der den bei einer Komprierung des Bandes in einem Trichter entstehende Staudruck misst.

20. Vorrichtung nach einem der Ansprüche 14 bis 19, dadurch gekennzeichnet, dass die beiden Sensoren ($S_1$,$S_2$) mit mechnischem und pneumatischem Messvorgang in Richtung der Faserbandbewegung in beliebiger Reihenfolge aufeinander folgen können und beliebig voneinander beabstandet sein können.

EP 0 431 481 A1

$\longrightarrow$ F

| $S_1$ | $S_2$ | $S_3$ | $S_U$ | L |

$f_1(X,Y,Z,U,V_1,M_1)$    $f_2(X,Y,Z,U,V_2,M_2)$    $f_3(X,Y,Z,U,V_3,M_3)$

$U(t)$

$f_{1E}(U)$
$f_{2E}(U)$
$f_{3E}(U)$

K | $f_{1E}(V_1,M_1)$    $f_{2E}(V_2,M_2)$    $f_{3E}(V_3,M_3)$

$f_{1K}(X,Y,Z)$    $f_{2K}(X,Y,Z)$    $f_{3K}(X,Y,Z)$

$\alpha_1,\alpha_2,\alpha_3$
$\beta_1,\beta_2,\beta_3$
$\gamma_1,\gamma_2,\gamma_3$

V |
$$df = f(t_2) - f(t_1)$$
$$df = \alpha dX + \beta dY + \gamma dZ$$
$$\Delta f(t) = \ddot{f}(t) - f_S$$

$f_{1S}, f_{2S}, f_{3S}$
$dX_S, dY_S, dZ_S$

VS

$\Delta f_{1K}, df_{2K}$    $\Delta f_{2K}, df_{2K}$    $\Delta f_{3K}, df_{3K}$

$dX_l(t)$    $dY_l(t)$    $dZ_l(t)$
$dX_k(t)$    $dY_k(t)$    $dZ_k(t)$

FIG.1

$\longrightarrow$ F

| $S_1$ | $S_2$ | $S_U$ | L |

$f_1(X,U,V_1,M_1)$         $f_2(X,Y,U,V_2,M_2)$         $U(t)$

$f_{1E}(V_1,M_1)$
$f_{2E}(V_1,M_1)$

$f_{1E}(U),\ f_{2E}(U)$

$X(t)$         $f_{2K}(X,Y)$

$\alpha,\beta$

$dX = X(t_2) - X(t_1)$

$\Delta X(t) = X(t) - X_S$

$df_{2K} = f_{2K}(t_2) - f_{2K}(t_1) = \alpha dX + \beta dY$

$X_S,\ dY_S$

VS

$X(t)$         $dY_l(t)$

$dX_l(t)$

$dX_k(t)$         $dY_k(t)$

$\Delta X(t)$

FIG. 2

EP 0 431 481 A1

FIG. 3A

FIG. 3B

FIG.4

FIG.5

**Europäisches Patentamt**

# EUROPÄISCHER RECHERCHENBERICHT

Nummer der Anmeldung

## EP 90 12 2911

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | EP-A-0 192 835  (MASCHINENFABRIK RIETER AG)<br>* Zusammenfassung; Abbildung 3 *<br>- - - | 14,15,17, 19 | G 01 N<br>33/36 |
| D,A | CH-A-6 295 46  (MASCHINENFABRIK RIETER AG)<br>* Zusammenfassung; Ansprüche 1-2; Abbildung 3 *<br>- - - | 1,14,18, 19 | |
| A | FR-A-2 556 747  (ZELLWEGER USTER S.A.)<br>* Zusammenfassung *<br>- - - | 1 | |
| A | BE-A-7 654 21  (DEUTSCHE AKADEMIE DER WISSEN-SCHAFTEN ZU BERLIN)<br>* Anspruch 1; Abbildung 3 *<br>- - - - - | 1,14 | |

| RECHERCHIERTE SACHGEBIETE (Int. Cl.5) |
|---|
| G 01 N |

Der vorliegende Recherchenbericht wurde für alle Patentansprüche erstellt

| Recherchenort | Abschlußdatum der Recherche | Prüfer |
|---|---|---|
| Den Haag | 26 Februar 91 | BAROCCI S. |